# EUROPEAN PATENT APPLICATION

(11) **EP 1 536 227 A2**
(43) Date of publication of application: **01.06.2005**
(21) Application number: 04012222.8
(22) Date of filing: 24.05.2004
(51) Int. Cl.: G01N 29/02, G01N 33/543, B81B 3/00, B01L 3/00, G01N 27/00

(54) **Quantitative biopolymer detecting system using monolithic piezoelectric cantilever by resonant frequency shift, method for fabricating the same system and method for detecting biopolymer quantitatively using the same system**

(30) Priority: 25.11.2003 KR 2003084160
(71) Applicant: Korea Institute of Science and Technology, Seoul (KR)
(72) Inventor: Kim, Tae Song, Mapo-Gu Seoul (KR); Hwang, Kyo Seon, Gyeonggi-Do (KR); Park, Jae Bum, Seodaemoon-Gu Seoul (KR); Lee, Jeong Hoon, Nowon-Gu Seoul (KR)
(74) Representative: Kampfenkel, Klaus, Dipl.-Ing.

(57) **Abstract**

A method for detecting a small amount of biopolymer by using resonant frequency shift of PZT monolithic cantilever system using a cantilever includes: an infinitesimal fluid transfer system having an inlet for allowing a reactant to be injected therethrough and an infinitesimal introduction channel for connecting the inlet and a reaction chamber; and a cantilever sensor installed in the reaction chamber and having a cantilever with one end fixed at a substrate, a piezoelectric capacitor for self-sensing and actuating on at least one side of an upper surface and a lower surface of the cantilever including a piezoelectric film, a lower electrode formed at a lower surface of the piezoelectric film and an upper electrode formed at an upper surface of the piezoelectric film, an electric pad for applying electricity to the lower electrode and the upper electrode, and a molecular recognition layer formed at at least one surface of the cantilever and so as to interact to an target biopolymer. Actuating and sensing can be applied to the cantilever using monolithic PZT cantilever without using an additional external actuator, so that the size of the cantilever sensor can be considerably reduced and coupled to a fine fluid transfer system to measure a very small amount of biopolymer.

## Description

### BACKGROUND OF THE INVENTION

### 1. Field of the Invention

The present invention relates to an analyzing system for sensing a small amount of protein and, more particularly, to an analyzing system using a cantilever type sensor using resonant frequency shift realizing reduced size thereof, sensing a protein in a liquid as well as in the air, successively. In addition, the present invention also relates to a sensing chip for sensing various biopolymer such as DNA, protein, enzyme or a cell.

### 2. Description of the Background Art

A quartz crystal mass balance (QCM) is a conventional system for sensing an analyte electrically or optically, which is, however, not suitable for a mass production because a single quartz crystal is too brittle to be formed small and thin.

Sensors using a cantilever under the researches measure a static deflection to detect a change in a surface of the cantilever(e.g. mass change thereof) caused by heat or gas adsorption to the surface radiating a light source(e.g. laser).

The sensing method measuring static deflection in order to sense biopolymer has been applied by Majumdar in Berkeley University in the United States and Fritz in an IBM Swiss Zurich research center. And a method to sense protein and gene using a biological reaction on a surface of a micro cantilever is published in the Nature Biotechnology 19, 856-860 (2001) and Science 288, 316-318 (200).

The sensing method by measuring the static deflection can be realized by focusing light source(e.g. laser) on the surface of the cantilever and collecting a position sensitive diode. In this case, there is a limit of difficulty to form a small-sized system because the sensing method requires a constant temperature device to minimize a motion of the microcantilever caused by an external heat and an optical space to estimate a deflection precisely.

There is another sensing method using a change of a resonant frequency. Thundat et al. of Oak Ridge national lab reveals in *Applied Physics Letters 80, 2219-2221 (2002)* that spring constant is changed when Na+ ion is adsorbed onto a surface of the microcantilever from measuring a resonant frequency. Some researchers including those in IBM Swiss Zurich research center have reported that a specific gas in the air can be sensed by measuring a resonant frequency thereof. However, in these cases, an additional actuator separate from the cantilever is required to attain the resonance and this causes a problem that the size of a chamber (i.e. a space required for a surface reaction with a sample to be sensed by the microcantilever) is inevitably increased, so that it becomes difficult to implement a diagnosis chip or an analyzing unit reacting with the small amount of in the range between a few pico litter and scores of micro litter.

Meanwhile, Microarrays, the conventional chips for the detection of biopolymer such as DNA or protein, have been suggested that the formation of several gold arrays on a substrate such as silicon wafer in order to form an organic molecular film thereon recognizing bio-bodies, and perform a surface modification, and interacts the target biopolymer. After interaction, the interacted biopolymer is scanned by laser, and then, fluorescent light is emitted owing to an attached fluorescent substance. The fluorescent light can be detected by using an optical sensing device (or detector). This method also has demerits in needs of bulky light source and the detector and labeling process, thereby making it difficult to obtain a compact size.

### SUMMARY OF THE INVENTION

Therefore, an object of the present invention is to provide a detection system of a small amount of protein related to disease using piezoelectric monolithic cantilever system by resonant frequency shift even in a liquid as well as in the air.

These and other objects of the invention are accomplished by a sample apparatus and a method for detecting and measuring a small amount of biopolymer such as DNA, protein, enzyme or a cell. To achieve these and other advantages and in accordance with the purpose of the present invention, as embodied and broadly described herein, there is provided a biopolymer detection system using a cantilever comprising: an infinitesimal fluid transfer system including an inlet for allowing a reactant to be injected therethrough, and an infinitesimal introduction channel for connecting the inlet and a reaction chamber; and a cantilever sensor embeded in the reaction chamber and including a cantilever with one end fixed at a substrate, a piezoelectric thin film for self-actuating and sensing deposited on at least one side of an upper surface and a lower surface of the cantilever, a lower electrode formed at a lower surface of the piezoelectric film and an upper electrode formed at an upper surface of the piezoelectric film, an electric pad for supplying electric current to both the lower electrode and the upper electrode, and a molecular recognition layer formed at at least one upper surface of the cantilever and the driving film so as to react to an analysis material.

To achieve the above object, there is also provided a method for fabricating an bioploymer detection system using a cantilever, comprising: a step of forming a cantilever sensor including a cantilever by using an MEMS technique, forming a actuating capacitor by deposition of piezoelectric film at a surface of the cantilever and de upper and lower electrodes at upper and lower surfaces of the piezoelectric film, deposition of an electronic pad at certain portions of the upper electrode and the lower electrode, and forming a molecular recognition layer on at least one side of the cantilever and the actuating capacitor; a step of forming upper and lower molds to form an inlet, a reaction chamber and an infinitesimal introduction channel for connecting the inlet with the reaction chamber; a step of putting a dissolved mold material in the molds and solidifying the mold material to form upper and lower plates; a step of fixing the cantilever sensor in the reaction chamber; and a step of bonding the upper and lower plates.

The foregoing and other objects, features, aspects and advantages of the present invention will become more apparent from the following detailed description of the present invention when taken in conjunction with the accompanying drawings.

### BRIEF DESCRIPTION OF THE DRAWINGS

The accompanying drawings, which are included to provide a further understanding of the invention and are incorporated in and constitute a part of this specification, illustrate embodiments of the invention and together with the description serve to explain the principles of the invention.

In the drawings:
Figures 1 to 3 illustrate structures of one embodiment of the present invention, in which:
   Figure 1 is a projective perspective view of an element detecting system using a cantilever in accordance with one embodiment of the present invention;
   Figure 2 is a perspective view of a cantilever sensor of Figure 1; and
   Figure 3 is a sectional view taken along line III-III of Figure 2; Figures 4 and 5 illustrate a result of sensing a micro element in the air, in which:
   Figure 4 is a graph showing a resonant frequency in the air; and
   Figure 5 is a graph showing a quantitative analysis result of CRP (C Reactive Protein) in the air by using a method for detecting micro element in accordance with one embodiment of the present invention; and
   Figures 6 to 8 are graphs showing an analysis result in a liquid by a cantilever type of the element detecting system with a cantilever.

### DETAILED DESCRIPTION OF THE PREFERRED EMBODIMENTS

Reference will now be made in detail to the preferred embodiments of the present invention, examples of which are illustrated in the accompanying drawings.

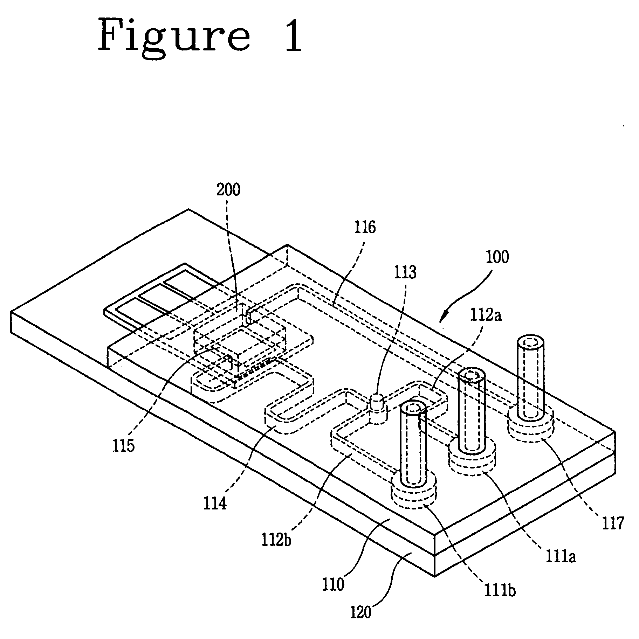

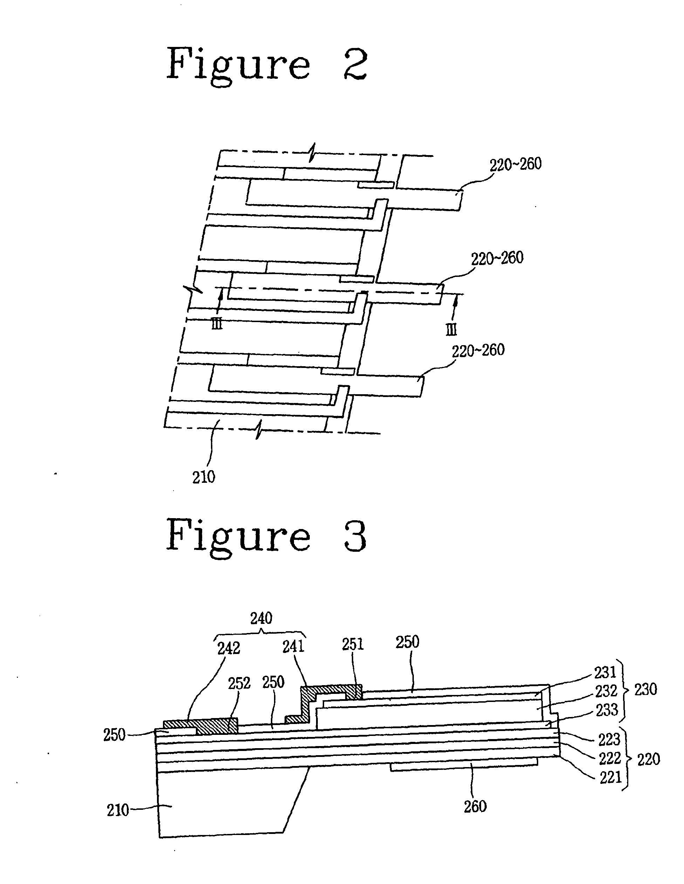

Figures 1 to 3 illustrate structures of one embodiment of the present invention, in which: Figure 1 is a projective perspective view of an element detecting system using a cantilever in accordance with one embodiment of the present invention, Figure 2 is a perspective view of a cantilever sensor of Figure 1, and Figure 3 is a sectional view taken along line III-III of Figure 2.

As shown in these drawings, the present invention provides an element detecting system comprising an infinitesimal fluid transfer system 100, and a cantilever sensor 200 fixed in a reaction chamber 115 of the infinitesimal fluid transfer system 100.

The infinitesimal fluid transfer system 100 is formed by combining an upper plate 110 with lower plate 120. The upper plate 110 includes inlets 111 a and 111b, an outlet 117 formed penetrating the upper plate 110, a mixing chamber 113 and the reaction chamber 115 formed insertedly and non-penetratingly at the surface of the upper plate 110 facing the lower plate, and inlet channel channels 112a, 112b and 114 and an inlet channel 116 formed insertedly at the surface of the upper plate 110 facing the lower plate 120.

The inlet channels 112a, 112b and 114 include entrance channels 112a and 112b connecting the inlets 111a and 111b and the mixing chamber 113, and a mixing channel 114 connecting the mixing chamber 113 with the reaction chamber 115. The outlet channel 116 connects the reaction chamber 115 with the outlet 117.

The mixing channel 114 is connected to a lower end of the reaction chamber 115(i.e. end near the lower plate 120), and the outlet channel 116 is connected to an upper end of the reaction chamber 115.

In the embodiment of the present invention shows that all the channels and the chambers are formed at the upper plate 110, but the invention includes that they are formed at the lower plate 120 or at both sides of the upper and lower plates 110 and 120.

The upper plate 110 and the lower plate 120 can be made of a polymer material (e.g. PDMS(Polydimethyl Siloxane), polycarbonate), or a glass material (e.g. Pyrex, quartz).

The cantilever sensor 200 includes a cantilever 220 with one end fixed at a substrate 210, a driving film 230 stacked at an upper surface of the cantilever 220; an insulation film formed to cover electric pads 241 and 242 for supplying electricity to the driving film 230, an electric signal pad (not shown) for detecting an electric signal, and the driving film 230 to prevent occurrence of conduction in a liquid, and a molecular recognition layer 260 formed at a lower surface of the cantilever 220 to react to an analysis material.

The cantilever 220 is formed as a triple layer consisting of a silicon oxide film 221, a silicon nitride film 222 stacked on the silicon oxide film 221 and a silicon oxide film 223 stacked on the silicon nitride film 222. In this case, the cantilever 220 is not necessarily formed as the triple layer but formed as a double layer of the silicon oxide film or silicon nitride film and silicon or formed as a single layer of silicon or silicon nitride film.

The driving film 230 includes a lower electrode 233 stacked at an upper surface of the cantilever 220, a piezoelectric film 232 stacked on the lower electrode and an upper electrode 231 stacked at an upper surface of the piezoelectric film 232.

The insulation film 250 is made of an inorganic material such as SiOx by using a PECVD(Plasma Enhanced Chemical Vapor Deposition), APCVD(Atmospheric Pressure Chemical Vapor Deposition) or evaporation device, or formed by evaporating a film made of an organic material such as parylene. An upper electrode opening 241 and a lower electrode opening 242 are formed to connect the electric pad 240 and the upper electrode 231 and lower electrode 233.

The electric pads includes an upper electric pad 241 connected to the upper electrode 231 and a lower electric pad 242 connected to the lower electrode 233. The electric pads are also connected to both the upper electrode 231 and the lower electrode 233. The electric pads are to detect a resonant frequency as an electric signal, and an optically transparent window is formed in the infinitesimal fluid transfer system in order to optically detect the electric signal.

The molecular recognition layer 250 is formed by fixing a reacting material to a measurement-subject material after modifying a self assembled monolayer (SAM) at the lower surface of the cantilever 220 or fixing a reacting material to the measurement-subject material after depositing Cr and Au at the lower surface of the cantilever 220 and modifying a calixcrown SAM thereon. For example, a material such as prostate specific antibody can be fixed on the SAM. However, without being restricted thereto, various materials can be fixed on the SAM according to the kind of a measurement-subject material.

As shown in Figure 2, in the cantilever sensor, more than two cantilevers 220 are formed on one substrate 210, and the driving film, the insulation film 250, the electrode pad 240, the electric signal pad (not shown) and the molecular recognition layer 260 are formed on each cantilever 220. In this respect, in order to obtain a reference resonant frequency, the molecular recognition layer 260 may not be formed on some cantilever 220.

A method for fabricating the element detecting system using a cantilever constructed as described above, includes a step of forming a cantilever sensor by using an MEMS technique; a step of forming upper and lower plates of the infinitesimal fluid transfer system; and a step of fixing the upper and lower plates and the cantilever sensor.

In the step of forming the cantilever sensor, the driving film, the electrode pad and the molecular recognition layer are formed by repeating deposition and etching on the substrate by using the MEMS technique.

In the step of forming the upper and lower plates, after molds are formed, which forms the inlets 111a and 111b, the outlet 117, the channels 112a, 112b, 114 and 116, the mixing chamber 113 and the reaction chamber 115, a dissolved mold material is put in the molds and solidified to form the upper and lower plates.

In the step of fixing the upper and lower plates and the cantilever sensor, the cantilever sensor 200, as formed in the step of forming the cantilever sensor, is fixed to the reaction chamber 115 in the following manner: The substrate 210 is fixed between the upper plate 110 and the lower plate 120 such that the cantilever lever 220 can be protruded in the reaction chamber 115, and then, the upper plate 110 and the lower plate 120 are bonded, wherein the preferable mold material is a PDMS, and the mold is Preferably made of die steel, Teflon or silicon.

The element detecting using the cantilever in accordance with the present invention operates as follows.

When different reactants are injected to the inlets 111a and 111b, these reactants are mixed while passing the mixing chamber 113 and the mixing channel 114 through the inlet channels 112a and 112b. As the mixture is filled up to the upper portion of the reaction chamber 115 through the mixing channel 114, it passes the outlet channel 116 and externally discharged through the outlet 117.

Meanwhile, a specific material of the mixture filled in the reaction chamber 115 is fixed at the molecular recognition layer 260 of the cantilever sensor 200. Thereafter, when electricity is supplied to electric pads 251 and 252 while changing a frequency, the piezoelectric film 232 is bent vertically, and at this time, a resonant frequency of the cantilever 220 is sensed through the electric signal pad.

Thereafter, it is possible to obtain a small amount of biopolymer fixed at the molecular recognition layer 260 by comparing the sensed resonant frequency with resonant frequency measured when the reaction material is not fixed to the molecular recoginition layer 260.

The element detecting system using a cantilever in accordance with the present invention has the following advantages.

Firstly, thanks to the driving film formed as the piezoelectric film, an actuator device is not additionally required, and thus, an overall size of the system is considerably reduced.

Secondly, by coupling the element detecting system to an infinitesimal transfer system, a desired reaction material can be sensed easily by using a very small amount of sample whose scale is from a few nano liter up to scores of micro liter.

Thirdly, since the resonant frequency is sensed by the electric pad, an additional optical space is not required. In this respect, however, the modification of the present invention proposes a method for measuring the resonant frequency by forming an optically transparent window.

Moreover, by forming several cantilevers on one substrate 210, one cantilever is used for frequency measurement and the others are used for measuring a sensing frequency. Thus, it is possible to reduce not only an error occurrence probability due to several recognitions, but also the number of obtaining resonant frequency. In addition, the molecular recognition layer is made of a different material in each cantilever 220, so that several materials can be detected at one time.

The method for detecting a micro element with the element detecting system using a cantilever is described below.

First, a method for detecting a small amount of biopolymer in a solution includes: a first step of injecting a cleaning solution such as a PBS solution into the inlets 111a and 111b to fill the reaction chamber 115; a second step of supplying electricity to the electric pads 251 and 252 to obtain a reference resonant frequency of the cantilever; a third step of supplying an analysis solution toward the inlets 111 a and 111 b and maintaining the state so as for the analysis solution to react to the molecular recognition layer 260; a fourth step of injecting a cleaning solution into the inlets 111 a and 111 b to fill the chamber 115; a fifth step of applying electricity to the electric pads 251 and 252 to obtain a resonant frequency of the cantilever; and a step of comparing the reference resonant frequency obtained in the second step with the resonant frequency obtained in the fifth step, in order to analyze a specific material in the analysis solution.

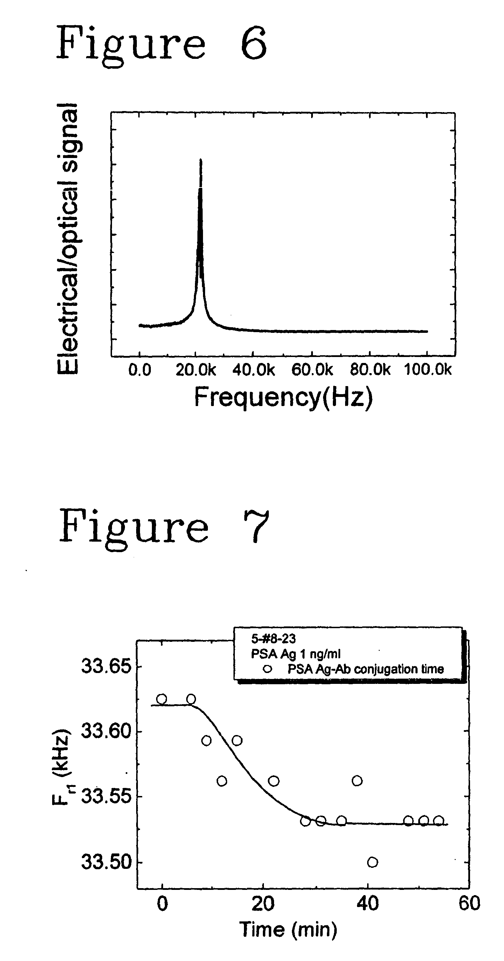

Figures 6 to 8 are graphs showing an analysis result in a liquid by using the element detecting system using a cantilever.

Figure 6 is a graph showing a result of the resonant frequency obtained in the second step. Accordingly, it is noted that the small amount of biopolymer detecting system using a cantilever in accordance with the present invention can obtain a resonant frequency even in a liquid.

Figure 7 is a graph showing a result of detection of mass of prostate cancer in a liquid by using PSA (Prostate Specific Antibody) serum.

With reference to Figure 7, after an analysis solution was injected into the inlets 111a and 111b to fill the reaction chamber, electricity was supplied to the electric pad at every predetermined period to obtain a resonant frequency of the cantilever. It's the result obtained from a patient of prostate cancer having a 1ng/ml PSA antigen density by diluting serum of the patient. From the result, it was possible to analyze the PSA having the density of 1ng/ml, which shows that proteins in serum, blood or blood plasma can be measured in the range of a few ng or pg.

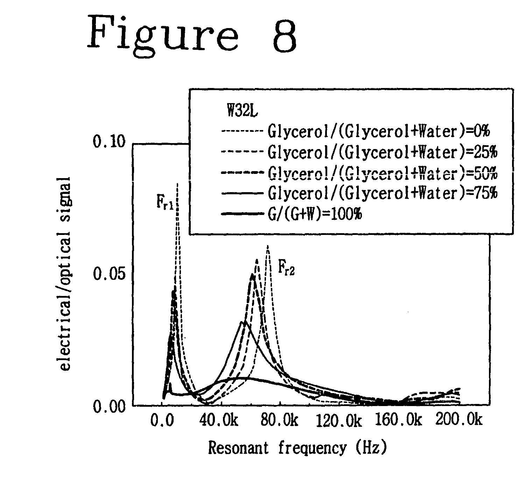

Figure 8 shows a result of measurement of viscosity and density of a fluid by using the element detecting system using a cantilever in accordance with the present invention. It is noted that a resonant frequency is being changed according to a change in a content of water and glycerol, that is, viscosity and density of a liquid. This result shows that it can be applied as a blood detecting sensor that can determine a degree of an illness through detection of an electrical resonant signal according to a fine change in viscosity of blood. Accordingly, by measuring viscosity and density of serum or blood, decrease or increase of red blood cells can be detected, according to which a degree of an illness can be determined.

A method for detecting a micro material in the air includes: a first step of injecting a cleaning solution into the inlets 111a and 111b to fill the chamber 115; a second step of injecting nitrogen gas into the inlets 111 a and 111 b to remove the cleaning solution from the chamber 115; a third step of supplying electricity to the electric pads 251 and 252 to obtain a reference resonant frequency of the cantilever; a fourth step of supplying an analysis solution toward the inlets 111a and 111 b and maintaining the state for a predetermined time so as for the analysis solution to react to the molecular recognition layer 260; a fifth step of injecting a cleaning solution into the inlets 111 a and 111 b to fill the chamber 115; a sixth step of injecting nitrogen gas into the inlets 111a and 111b to remove the cleaning solution from the chamber 115; a seventh step of applying electricity to the electric pads 251 and 252 to obtain a resonant frequency of the cantilever; and a step of comparing the reference resonant frequency obtained in the third step with the resonant frequency obtained in the seventh step.

The cleaning solution is preferably a PBS.

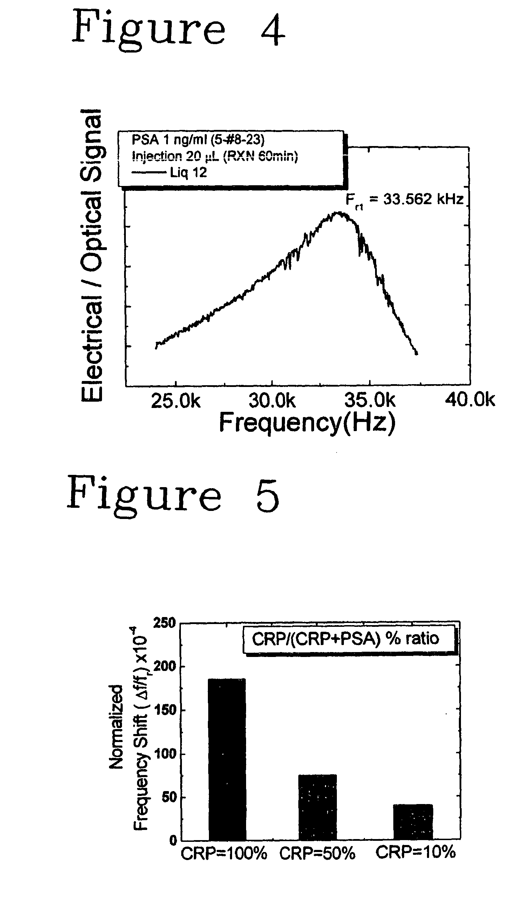

Figures 4 and 5 illustrate results of detecting a small amount of protein related to disease in the air. Specifically, Figure 4 illustrates a profile of the resonant frequency in the air, and Figure 5 illustrates a quantitative analysis result of CRP (C Reactive Protein) in the air by using the method for detecting a small mount of protein in accordance with the present invention.

As mentioned above, in the present invention, the resonant frequency can be measured even in a liquid as well as in the air, the element detecting system can be used as a sensor for detecting a biopolymer, as a humidity sensor by forming the molecular recognition layer with gelatin, as a mercury detecting sensor, as a high sensitive gas sensor, and as a weight sensor that is able to detect a weight from a few pico grams to a few micro grams.

Meanwhile, for the purpose of detecting a biopolymer such as protein or DNA, an antibody is formed on the molecular recognition layer, and then, in order not to change characteristics of the biopolymer such as protein or DNA, a temperature is not changed at a room temperature and only a pressure is lowered to gasificate the biopolymer, the detection subject, so that it can react to the molecular recognition layer.

As so far described, the present invention has various advantages as follows. In this respect, the present invention is considered established even when the following effects are not exerted.

That is, for example, first, by providing the cantilever using the piezoelectric film, vibration of a constant frequency can be applied to the cantilever without using an additional actuator. Thus, the size of the cantilever sensor can be considerably reduced and coupled to a fine fluid transfer system to measure a very small amount of reaction material.

In addition, by having the insulation film, a resonant frequency can be obtained even in a liquid, and a resonant frequency that is changed over time in the liquid can be obtained.

As the present invention may be embodied in several forms without departing from the spirit or essential characteristics thereof, it should also be understood that the above-described embodiments are not limited by any of the details of the foregoing description, unless otherwise specified, but rather should be construed broadly within its spirit and scope as defined in the appended claims, and therefore all changes and modifications that fall within the metes and bounds of the claims, or equivalence of such metes and bounds are therefore intended to be embraced by the appended claims.

## Claims

1. A method for detecting a small amount of biopolymer by using resonant frequency shift of PZT monolithic cantilever system comprising:
an infinitesimal fluid transfer system including an inlet for allowing a reactant to be injected therethrough, and an infinitesimal introduction channel for connecting the inlet and a reaction chamber; and
a cantilever sensor including a cantilever with one end fixed at a substrate, a piezoelectric thin film for self-actuating and sensing deposited on at least one side of an upper surface and a lower surface of the cantilever, a lower electrode formed at a lower surface of the piezoelectric film and an upper electrode formed at an upper surface of the piezoelectric film, and an electric pad for supplying electricity to both the lower electrode and the upper electrode, the cantilever sensor being installed in the reaction chamber.

2. The system of claim 1 further comprising
a molecular recognition layer formed at at least one surface of the cantilever so as to react to an target biopolymer.

3. The system of claim 1, wherein the infinitesimal transfer system has two or more inlets, and the inlet channel includes an entrance channel with one end connected to the inlets and a mixing channel with one end connected to a point where the inlet channels meet and the other end connected to the reaction chamber.

4. The system of claim 3 further comprises a mixing chamber formed at a point where the entrance channel and the mixing channel meet.

5. The system of claim 1, wherein the infinitesimal fluid transfer system further comprises:
an outlet formed to allow a reactant to be discharged; and
an outlet channel connecting the outlet with the reaction chamber.

6. The system of claim 5, wherein the outlet channel is connected to an upper end of the reaction chamber.

7. The system of claim 1, wherein the inlet channel is connected to a lower end of the reaction chamber.

8. The system of claim 1, wherein an optically transparent window is formed to enable an optical measurement into the reaction chamber from outside.

9. The system of claim 1, wherein the infinitesimal fluid transfer system is made of a polymer material.

10. The system of claim 9, wherein the polymer material is PDMS.

11. The system of claim 1, wherein the infinitesimal fluid transfer system is made of a glass material.

12. The system of claim 11, wherein the glass material is made of pyrex or quartz.

13. The system of claim 1, wherein an insulation film is formed to cover the piezoelectric monolithic cantilever in order to prevent conduction in a liquid.

14. The system of claim 13, wherein the insulation film is an inorganic insulator.

15. The system of claim 14, wherein the inorganic insulator is SiOx.

16. The system of claim 13, wherein the insulation film is an organic insulator.

17. The system of claim 16, wherein the organic insulator is parylene.

18. The system of claim 1 further comprising:
an electric signal pad connected to the upper electrode and the lower electrode and detecting an electric signal.

19. The system of claim 1, wherein two or more cantilevers are arrayed, and the self-actuating and sensing capacitor are formed at every cantilever.

20. The system of claim 2, wherein two or more cantilevers are arrayed, and the self-actuating and sensing capacitor , the electrode pad and the molecular recognition layer are formed at every cantilever.

21. The system of claim 1, wherein the piezoelectric capacitor is formed on a single layer of silicon or silicon nitride film.

22. The system of claim 1, wherein the piezoelectric capacitor is formed on a double layer of silicon nitride film and silicon.

23. The system of claim 1, wherein the piezoelectric capacitor is formed on a double layer of silicon oxide layer and silicon.

24. The system of claim 1, wherein the piezoelectric capacitor is formed on a triple layer of silicon oxide film, silicon nitride film and silicon oxide film.

25. The system of claim 2, wherein the biopolymer receptor was immobilized using an self assembled monolayers(SAMs) on cantilever for the detection of target biopolymer.

26. The system of claim 25, wherein Cr and Au are deposited at a surface of the cantilever, on which the SAM layer is formed.

27. A method for fabricating an element detecting system using a cantilever, comprising:
a step of forming a cantilever sensor comprising fabricating a cantilever by using an MEMS technique, forming a piezoelectric thin film for self-actuating and sensing deposited on at least one side of an upper surface and a lower surface of the cantilever, a lower electrode formed at a lower surface of the piezoelectric film and an upper electrode formed at an upper surface of the piezoelectric film, stacking an electronic pad at certain portions of the upper electrode and the lower electrode, and forming a molecular recognition layer at at least one side of the cantilever and the driving film;
a step of forming upper and lower molds to form an inlet, a reaction chamber and an infinitesimal introduction channel for connecting the inlet and the reaction chamber;
a step of putting a dissolved mold material in the molds and solidifying it to form upper and lower plates;
a step of fixing the cantilever sensor formed in the cantilever sensor forming step in the reaction chamber; and
a step of bonding the upper and lower plates.

28. The method of claim 27, wherein the mold material is PDMS.

29. The method of claim 28, wherein the mold is formed on die steel, teflon or silicon.

30. The method of claim 27, wherein the mold material is glass.

31. The method of claim 27 further comprising:
forming an insulation film to cover the monolithic piezoelectric cantilever after forming the electric pad.

32. A method for detecting a small amount of biopolymer by using resonant frequency shift of PZT monolithic cantilever system using a cantilever of claim 1 comprises:
a step of injecting a cleaning solution such as a PBS solution into the inlets to fill the reaction chamber;
a step of applying electricity to the electric pads to obtain a reference resonant frequency of the cantilever;
a step of supplying an analysis solution toward the inlets and maintaining the state so as for the analysis solution to react to the molecular recognition layer;
a step of injecting a cleaning solution into the inlets to fill the chamber;
a step of applying electricity to the electric pads to obtain a resonant frequency of the cantilever; and
a step of comparing the reference resonant frequency obtained in the second step with the resonant frequency obtained in the fifth step.

33. The method of claim 32, wherein the cleaning solution is a PBS solution.

34. In a method for detecting a micro material by using the element detecting system using a cantilever of one of claim 1 to 24, an analysis solution is injected into the inlet to fill the reaction chamber and electricity is applied to the electric pad at every predetermined time to thereby obtain a resonant frequency of the cantilever.

35. A method for detecting a small amount of biopolymer by using resonant frequency shift of PZT monolithic cantilever system using a cantilever of claim 1 comprises:
a step of injecting a cleaning solution into the inlets to fill the chamber;
a step of injecting nitrogen gas into the inlets to remove the cleaning solution from the chamber;
a step of supplying electricity to the electric pads to obtain a reference resonant frequency of the cantilever;
a step of supplying an analysis solution toward the inlets and maintaining the state for a predetermined time so as for the analysis solution to react to the molecular recognition layer;
a step of injecting a cleaning solution into the inlets to fill the chamber;
a step of injecting nitrogen gas into the inlets to remove the cleaning solution from the chamber;
a step of supplying electricity to the electric pads to obtain a resonant frequency of the cantilever; and
a step of comparing the reference resonant frequency obtained in the third step with the resonant frequency obtained in the seventh step.

36. The method of claim 35, wherein the cleaning solution is PBS.

37. A method for detecting a small amount of biopolymer by using resonant frequency shift of PZT monolithic cantilever system using a cantilever of claim 20, molecular recognition layers of each cantilever are surface-processed differently in order to detect several materials at one time.

38. In a method for measuring viscosity and density of a liquid by using the element detecting system using a microcantilever of claim 1, a liquid is injected into a reaction chamber, and then, a resonant frequency of the microcantilever and a change in the width are measured to measure viscosity and density of a liquid.

39. The method of claim 38, wherein the liquid is blood plasma, serum or blood, and decrease and increase of red blood cells is detected by measuring viscosity and density of blood plasma, serum or blood to thereby measure a degree of an illness.

40. In a method for sensing a material by using the element detecting system using a microcantilever of claim 2, a reaction material of the molecular recognition layer is in a gas state.

41. The method of claim 40, wherein the molecular recognition layer is formed by solidifying gelatin, and the gas is humidity.

42. In a method for sensing biopolymer by using the element detecting system using a microcantilever of claim 2, wherein the molecular recognition layer includes an antibody reacting to protein or DNA or complementary DNA, and the reaction material such as protein or DNA is gasificated by lowering a pressure in a room temperature, so as to react to the molecular recognition layer.
